# FASCICULE DE BREVET EUROPEEN

(11) **EP 0 672 404 B1**
(45) Date de publication et mention de la délivrance du brevet: **24.04.1996**
(21) Numéro de dépôt: 95400187.1
(22) Date de dépôt: 27.01.1995
(51) Int. Cl.: A61K 7/02

(54) **Gel aqueux de maquillage à organopolysiloxane**
Wässriges make-up Gel mit Organopolysiloxane
Organopolysiloxane containing aqueous make-up gel

(30) Priorité: 14.03.1994 FR 9402944
(43) Date de publication de la demande: 20.09.1995
(73) Titulaire: L'OREAL, F-75008 Paris (FR)
(72) Inventeur: Bara, Isabelle, F-75014 Paris (FR); Mellul, Myriam, F-94240 L'Hay-les-Roses (FR)
(74) Mandataire: Dodin, Catherine

(56) Documents cités:
- EP-A- 0 540 357
- WO-A-92/09263
- FR-A- 2 686 510

## Description

L'invention a pour objet un gel aqueux à organopolysiloxane utilisable dans le domaine cosmétique pour le maquillage du visage et/ou du corps, en particulier se présentant sous forme d'un fond de teint ou d'un fard à joues. L'invention se rapporte aussi à un procédé de maquillage consistant à appliquer ce gel sur le visage.

On utilise parfois pour le maquillage de la peau des gels aqueux constitués d'une phase hydrophile comportant des agents gélifiants formés essentiellement de polymères hydrophiles ces gels aqueux ne comportent pas de phase grasse. Ils peuvent en outre être colorés par incorporation de colorants et/ou pigments hydrophiles et apparaissent opaques ou translucides. Ces gels servent, en particulier, à la préparation de fonds de teint ou de fards à joues ou à paupières.
Ces gels aqueux colorés translucides sont connus pour leur apport de "bonne mine" et leur fraîcheur à l'application ; l'utilisateur de tels gels n'a pas l'impression d'être réellement maquillé.
Ces gels classiques ont l'inconvénient de procurer un inconfort aux personnes à peau sèche en raison de l'absence de corps gras et de provoquer un effet collant et tenseur de la peau du fait de la présence d'agents gélifiants.
En outre l'application de ces gels est souvent difficile du fait d'un manque d'effet glissant et d'un séchage trop rapide ; ces gels confèrent à la peau une hétérogénéité de maquillage, c'est-à-dire des zones claires et foncées.
Pour remédier à l'inconfort de ces gels, on est conduit à incorporer dans ces derniers des hydratants comme, par exemple des polyols, tels que la glycérine, mais l'effet collant conféré à la peau s'en trouve alors accentué.
Ces inconvénients rendent donc l'utilisation de ces gels malaisée, voire rédhibitoire.
Il subsiste donc le besoin d'un gel de maquillage d'application aisée, qui glisse et s'étale bien, non collant, qui ne sèche pas trop vite et qui confère à la peau un maquillage homogène.

La demanderesse a maintenant trouvé un gel aqueux fluide de maquillage, permettant d'atteindre ces objectifs.
Selon l'invention, ce gel aqueux contient au moins un polymère hydrophile comme gélifiant et une matière colorante dispersible ou soluble dans la phase aqueuse, et comprend en outre au moins un organopolysiloxane solubilisé dans la phase aqueuse.

Certes, on connaît, par le document WO-9209263, des gels de soin aqueux contenant de la glycérine en tant qu'humectant hydrosoluble, un polymère gélifiant hydrophile et un mélange de gomme de silicone dans une silicone liquide sous forme de macro-dispersion. Toutefois, ces gels sont troubles du fait de la dispersion hétérogène et macroscopique de gomme et de silicone liquide. Les globules huileux dispersés dans le gel ont un diamètre moyen de plusieurs µm.

Ce gel, qui reste stable grâce à sa forte viscosité de l'ordre de 4-300 Pa.s (40 à 3000 poises) casse lorsqu'on l'applique sur la peau, libérant des "sacs d'huile". Il s'ensuit un aspect hétérogène immédiatement visible, ce qui n'est pas sans présenter quelques problèmes dans le cas où l'on envisage son utilisation pour le maquillage.

Par contre, le gel conforme à l'invention convient parfaitement bien au maquillage. Il confère à la peau un aspect très naturel, une bonne mine, un aspect non gras et un toucher non collant. Il s'applique facilement sur la peau, sans pelucher ; il glisse bien et ne sèche pas trop rapidement. Le maquillage résultant est parfaitement homogène. Les propriétés cosmétiques du gel selon l'invention sont donc supérieures à celles des gels classiques.
Ces multiples avantages destinent le gel de l'invention au maquillage de tout type de peaux, et même des peaux sèches.

La teneur en organopolysiloxane solubilisé dans le gel aqueux de l'invention peut être choisie de 0,02 % à 50 % en poids par rapport au poids total du gel et dépend du type d'organopolysiloxane utilisé.
Au dessus de 50 % d'organopolysiloxane, la composition confère un aspect poisseux à la peau.
Les organopolysiloxanes utilisables dans l'invention peuvent être rendus solubles soit par modification chimique, soit de façon physique par adjonction d'un composé tel qu'un tensioactif. lls peuvent être utilisés seuls ou en mélange.

En particulier, comme organopolysiloxane solubilisé chimiquement, on peut utiliser un copolymère de siloxane et de protéine, hydrolysée ou non, comme décrit notamment dans le document EP-A-540 357.
Des exemples de tels copolymères sont les copolymères de polysiloxane ou dérivés liés de manière covalente par greffage à une protéine hydrolysée ou non telle que la caséine, I'élastine, le collagène, la kératine, la soie ou une protéine de blé ou de soja.
On peut par exemple utiliser un siloxane phosphaté greffé sur une protéine de blé hydrolysée. Ces copolymères greffés sont notamment vendus par la société CRODA sous la dénomination Crodasone W, par la société PHOENIX sous la dénomination Pecosil SWP 83 ou la dénomination Non-P-Silicon Protein.
Les teneurs en copolymères de siloxane et de protéine, hydrolysée ou non, sont de préférence choisies de 1 % à 50 % et, de manière encore plus préférée, de 10% à 50% en poids par rapport au poids total du gel.

Comme autre organopolysiloxane solubilisé chimiquement, utilisable dans le gel de l'invention, on peut également citer les diméthicones copolyols (nomenclature CTFA) et leurs dérivés.

Des diméthicones copolyols ont en particulier été présentés par la société DOW CORNING lors du 17ème congrès international de l'I.F.S.C.C. d'octobre 1992 et rapportés dans l'article "water-soluble dimethicone copolyol waxes for personal care industry" de Linda Madore et al., pages 1 à 3.
Ces diméthicones copolyols sont des polydiméthylsiloxanes (PDMS) comportant une ou plusieurs fonctions éthers, solubles dans l'eau (oxyalkylène, notamment oxyéthylène et/ou oxypropylène).
De tels diméthicones copolyols sont notamment vendus par la société GOLDSCHMIDT sous la dénomination ABIL B8851 ou ABIL B88183.
On peut citer aussi les composés KF 351 à 354 et KF 615 A vendus par la société SHIN ETSU ou la DMC 6038 de la société WACKER.
Les dérivés de diméthicones copolyols utilisables dans l'invention sont en particulier les diméthicones copolyols à groupement phosphate, sulfate, chlorure de myristamide propyldiméthylammonium, stéarate, amine, glycomodifié, etc.
On utilise comme dérivés de diméthicones copolyols notamment les composés vendus par la société SILTECH sous la dénomination Silphos A100, Siltech amine 65, Silwax WDIS, myristamido silicone quat, ou par la société PHOENIX sous la dénomination Pecosil PS 100.
On peut utiliser également les dérivés vendus par la société WACKER sous la dénomination VP 1661, ou par la société DOW CORNING sous la dénomination 2501 cosmetic wax.
On utilise les diméthicones copolyols et leurs dérivés dans la composition conforme à l'invention à des concentrations de préférence choisies de 0,5 % à 20 % en poids par rapport au poids total du gel.

Comme organopolysiloxane solubilisé physiquement, utilisable dans l'invention, on peut citer des organopolysiloxanes solubilisés à l'aide d'un tensioactif de valeur HLB supérieure à 8, de préférence supérieure ou égale à 10 ; comme cela est bien connu, on entend par HLB (Hydrophilic-Lipophilic Balance, en terminologie anglo-saxonne ou équilibre hydrophile-lipophile) l'équilibre entre la dimension et la force du groupe hydrophile et la dimension et la force du groupe lipophile de l'agent tensioactif. Ces organopolysiloxanes se présentent sous forme d'émulsion ou de microémulsion contenant de 10 % à 90 % en poids d'organopolysiloxane par rapport au poids total de l'émulsion ou microémulsion.
Selon l'invention, la taille moyenne des particules d'organopolysiloxane dispersées est de l'ordre de 1 µm ce qui permet d'obtenir un maquillage très homogène de la peau.
De préférence, ces organopolysiloxanes peuvent être des polydiméthylsiloxanes (PDMS) éventuellement de type gomme, seuls ou en mélange avec des cyclométhicones.
On utilise ces organopolysiloxanes solubilisés physiquement à des teneurs choisies de préférence de 0,02 % à 20 % en poids par rapport au poids total du gel ; même à une teneur d'organopolysiloxanes supérieure à 10 % en poids, le gel de maquillage de l'invention reste stable, ce qui n'est pas le cas de celui objet du document WO-9209263.
Des exemples d'organopolysiloxane solubilisés physiquement utilisables dans l'invention sont notamment les SM2140 ou SM2112 de DOW CORNING, la MFF 5015-70 de SILTECH, le TP 503 ou le TP 233 B de UNION CARBIDE.
Les tensioactifs de valeur de HLB (équilibre hydrophile/lipophile) supérieure à 8 sont notamment des éthers d'alcool gras en C₆ à C₂₄ et de polyoxyde d'éthylène. lls sont incorporés en général à raison de 0,5 % à 30 % en poids par rapport au poids total de l'émulsion ou microémulsion. Ce sont par exemple les polyoxyéthyléné 12 cétyl/stéaryl éther ou polyoxyéthyléné 20 cétyl/stéaryl éther.

Le gel conforme à l'invention a une consistance souple ou fluide.
Sa viscosité absolue, mesurée à 25 °C au viscosimètre EBBRECHT, a une valeur choisie de 1 poise à 100 poises (0,1 Pa.s à 10 Pa.s), et de manière préférée, une valeur de 5 poises à 30 poises (0,5 Pa.s à 3 Pa.s).
Il est tout à fait surprenant qu'avec des valeurs de viscosité aussi faibles que 1 poise (0,1 Pa.s) on puisse incorporer dans ce gel des organopolysiloxanes à des teneurs supérieures à 10 % en poids par rapport au poids total du gel.

La viscosité du gel de l'invention dépend de la nature et de la quantité du ou des polymères hydrophiles, utilisés en tant que gélifiants.
Ces gélifiants sont par exemple les polymères ou copolymères acryliques et polyméthacryliques comme les copolymères acide acrylique/acrylate d'éthyle et les polymères carboxyvinyliques. Des exemples de tels polymères ou copolymères sont notamment les "carbomer" (CTFA) vendus par la société GOODRICH sous la dénomination Carbopol ou le polyglycérylméthacrylate vendu par la société GUARDIAN sous la dénomination Lubragel ou encore le polyglycérylacrylate vendu sous la dénomination Hispagel par la société HISPANO CHIMICA ou enfin le mélange polyacrylamide/C₁₃-C₁₄ lsoparaffin/Laureth7 vendu par la société SEPPIC sous la dénomination Sepigel.
Comme autres gélifiants utilisables dans l'invention, on peut citer : les dérivés de polysaccharides, tels que les dérivés cellulosiques comme la carboxyméthylcellulose, l'hydroxyméthylcellulose, l'hydroxypropylcellulose, la cellulose microcristalline ; les polymères de xanthane, de gellane, de rhamsan, les alginates, la maltodextrine, l'amidon et ses dérivés, l'acide hyaluronique et ses sels, la gomme de karoya, la farine de caroube, les dérivés de guar notamment l'hydropropylguar. On peut encore utiliser comme polymère hydrophile les polyéthylèneglycols (PEG) et leurs dérivés, les polyvinylpyrrolidones et leurs dérivés.
De manière préférée, on utilise en tant que polymère hydrophile un dérivé acrylique, tel qu'un carbomer (Carbopol) que l'on neutralise, de manière classique, à l'aide d'un agent basique comme, par exemple la triéthanolamine ou la soude, un polyglycérylméthacrylate (Lubragel) ou des polysaccharides tels que des dérivés de cellulose.
Ces polymères hydrophiles sont généralement présents à raison de 0,01 % à 30 %, et de préférence de 0,1 % à 5 % en poids par rapport au poids total du gel.

Le gel de l'invention peut contenir, comme matières colorantes, des colorants organiques et/ou des pigments, de préférence hydrophiles ou rendus hydrophiles pour assurer leur dispersion dans le milieu.
Comme colorants organiques hydrophiles, on peut citer les sels solubles dans l'eau comme le sel disodique de ponceau, le sel disodique du vert d'Alizarine, le jaune de quinoléïne, le sel trisodique d'amarante, le sel disodique de tartrazine, le sel monosodique de rhodamine, le sel monosodique de résorcine, les verts de quinizarine, le sel trisodique de pyramine, le sel disodique de fuchine, la bétanine, la xanthophylle et le caramel. Lorsque le gel de maquillage ne comprend que ces colorants, il a un aspect transparent et plutôt translucide.
Comme pigments hydrophiles ou rendus hydrophiles, on utilise les pigments dispersibles dans l'eau comme notamment les oxydes métalliques dont la taille moyenne de particules est éventuellement nanométrique. Ces oxydes métalliques sont, par exemple, les oxydes de fer, de titane, de manganèse, de zinc, de chrome.
Comme autre pigment dispersible dans la phase aqueuse du gel de l'invention, on peut citer le pigment mélanique de granulométrie inférieure à 1 µm et plus particulièrement inférieure à 500 nm préparé selon le procédé décrit dans le document FR 93-04960.
Selon ce procédé, la mélanine d'origine naturelle ou synthétique est, dans une première étape, solubilisée à une température comprise entre 10°C et 50°C, dans une solution aqueuse contenant un agent alcalinisant comme par exemple la soude et/ou un agent séquestrant tel que notamment le sel de sodium de l'acide éthylène diamine tétra(méthylènephosphonique) puis, dans une deuxième étape, la mélanine est précipitée par addition d'un sel de métal alcalino-terreux. Le précipité de pigment mélanique ainsi obtenu est ensuite isolé, par exemple par filtration avant d'être lyophilisé.
Comme pigment mélanique d'origine synthétique on peut utiliser le pigment mélanique obtenu, de manière connue, par oxydation du 5,6-dihydroxyindole. Le pigment mélanique, lorsqu'il est présent dans le gel, est, de préférence associé à des oxydes métalliques comme ceux décrits ci-dessus, en particulier dans la préparation de fonds de teint.
Lorsque le gel contient des pigments il a un aspect opaque.
Les colorants ou pigments sont généralement présents à une teneur de 0,001 % à 10 % en poids par rapport au poids total du gel, et de préférence de 0,01 % à 1 % pour les colorants, et de 1 % à 10 % pour les pigments.

Selon l'invention, il est en outre possible d'ajouter au gel des additifs tels que ceux classiquement utilisés dans le domaine cosmétique.

L'additif peut être présent à raison de 0-30 % en poids par rapport au poids total du gel. Les quantités précises sont fonction du type d'additif choisi et sont à la portée de l'homme du métier.
Cet additif peut être un agent gélifiant autre qu'un polymère et par exemple, une silice hydrophile ou un silicate tel que le silicate d'aluminium et magnésium appartenant au groupe des montmorillonites telles que des hectorites et bentonites. L'additif peut également être un actif soluble dans l'eau tel que notamment un polyol (glycérol, propylèneglycol, butylèneglycol) pour ses propriétés hydratantes. Contrairement aux gels aqueux de l'art antérieur, les propriétés sensorielles du gel aqueux de l'invention ne sont nullement diminuées par la présence de polyol; il peut être utilisé à raison de 0,5 % à 20 % en poids par rapport au poids total du gel.
D'autres actifs hydratants solubles dans l'eau peuvent aussi être incorporés dans le gel de l'invention comme les chondroitines sulfatées, les dérivés de chitine, l'acide hyaluronique, les dérivés de protéines notamment de kératine, différents monoalcools (éthanol, isopropanol) en vue de conférer davantage de fraîcheur au gel lors de son étalement, ainsi que des sucres tels que le sorbitol, le glucose, le saccharose ; des oligo-éléments, des acides aminés, des vitamines, des filtres hydrophiles, de l'urée, de l'allantoïne.
Comme autre additifs, on peut encore citer les charges, les conservateurs solubles dans l'eau tels que la diazolidinylurée (Germal) ou le parahydroxybenzoate de méthyle (Paraben), les parfums, les agents neutralisants tels que la triéthanolamine ou la soude en vue d'ajuster le pH.

Ainsi, le gel selon l'invention comprend plus particulièrement, en poids :
- de 0,02 % à 50 % d'organopolysiloxane solubilisé dans la phase aqueuse,
- de 0,01 % à 30 % de polymère hydrophile,
- de 0,001 % à 10 % de matière colorante,
- de 0 % à 30 % d'additif, soluble ou dispersible dans l'eau,
et de l'eau en quantité suffisante pour avoir 100 %.

L'invention a aussi pour objet un procédé de maquillage d'un visage humain, consistant à appliquer sur le visage un gel tel que défini précédemment.

Le gel de l'invention est particulièrement bien adapté à la réalisation d'un fard à joues ou à paupières, ou d'un fond de teint.
Aussi, l'invention a encore pour objets un fard à joues ou à paupières, et un fond de teint, consistant en un gel aqueux tel que défini précédemment.

D'autres avantages et caractéristiques apparaîtront mieux dans les exemples qui suivent donnés à titre illustratif et non limitatif. Les quantités sont données en pourcentage pondéral.
Les gels illustrés ci-dessous sont stables dans le temps.

### EXEMPLE 1 : Fard à joues de couleur prune, foncé.

| | |
|---|---|
| . Carbopol 954 | 1,5% |
| . Triéthanolamine | 1,5 % |
| . Silivax WD-IS (*) | 5 % |
| . Polyvinylpyrrolidone | 1 % |
| . Polyéthylèneglycol 600 | 8,4 % |
| . Sel disodique de ponceau SX | 0,18 % |
| . Sel disodique du vert d'Alizarine G | 0,02 % |
| . Parahydroxybenzoate de méthyle | q.s. |
| . Eau | q.s.p. 100 % |

| | |
|---|---|
| (*) PDMS oxyéthyléné à groupements stéarate vendu par la Société Siltech. | |

Ce fard est préparé à température ambiante par mélange de ces constituants à l'aide d'un agitateur de type turbine Rayneri.
D'application aisée, ce gel confère, après étalement sur la peau, un maquillage homogène très naturel et une bonne mine. En outre, il présente une bonne tenue dans le temps.

### EXEMPLE 2 : Fond de teint de couleur caramel.

| | |
|---|---|
| . Carbopol 954 | 0,75 % |
| . Triéthanolamine | 0,8 % |
| . Silicone émulsion TP 503 (*) | 14,28 % |
| . Sel disodique de Ponceau SX | 0,18 % |
| . Sel disodique du vert d'Alizarine | 0,08 % |
| . Jaune de quinoléine | 0,14 % |
| . Parahydroxybenzoate de méthyle | q.s. |
| . Eau | q.s.p. 100 % |

| | |
|---|---|
| (*) TP 503 vendu par la Société Union Carbide : mélange de PDMS de haut poids moléculaire et de cyclométhicones en émulsion dans l'eau ; l'émulsion présente des particules de diamètre voisin de 1 µm. | |

Ce fond de teint est préparé dans les mêmes conditions que celles de l'exemple précédent. On obtient un gel très doux, ne peluchant pas, très frais à l'application et filmogène. Il s'applique de façon homogène et confère à la peau un léger hâle naturel.

### EXEMPLE 3: Fond de teint de couleur caramel

| | |
|---|---|
| . Carbopol 954 | 1,5 % |
| . Triéthanolamine | 2,5 % |
| . Silicone Non P Protein (*) | 10 % |
| . Sel disodique de Ponceau SX | 0,18 % |
| . Sel disodique du vert d'Alizarine | 0,08 % |
| . Jaune de quinoléine | 0,14 % |
| . Parahydroxybenzoate de méthyle | q.s. |
| . Eau | q.s.p. 100 % |

| | |
|---|---|
| (*) copolymère de silicone, avec le greffage d'une protéine de blé, dans l'eau à 35 % vendu par la Société Siltech. | |

Ce fond de teint est préparé comme dans l'exemple 1.
On obtient un maquillage conférant à la peau une "bonne mine", d'application facile, et de bonne tenue.

## Revendications

1. Gel de maquillage comprenant une phase aqueuse, un polymère hydrophile comme gélifiant, et une matière colorante soluble ou dispersible dans la phase aqueuse, caractérisé en ce qu'il comprend en outre au moins un organopolysiloxane solubilisé dans la phase aqueuse.

2. Gel selon la revendication 1, caractérisé en ce qu'il comprend, en poids, 0,02-50 % en poids d'organopolysiloxane solubilisé dans la phase aqueuse.

3. Gel selon l'une quelconque des revendications précédentes, caractérisé en ce qu'il présente une viscosité absolue d'une valeur de 0,1-10 Pa.s. (1-100 poises).

4. Gel selon l'une quelconque des revendications précédentes, caractérisé en ce qu'il présente une viscosité absolue de 0,5-3 Pa.s (5-30 poises).

5. Gel selon l'une quelconque des revendications précédentes, caractérisé en ce que l'organopolysiloxane solubilisé dans la phase aqueuse est choisi parmi les copolymères de siloxane et de protéine hydrolysée ou non; les diméthicones copolyols et leurs dérivés; les organopolysiloxanes solubilisés à l'aide d'un tensioactif de valeur de HLB (équilibre hydrophile-lipophile) supérieure à 8.

6. Gel selon la revendication 5, caractérisé en ce qu'il comprend, en poids, 1-50 % d'un copolymère de siloxane et de protéine.

7. Gel selon l'une quelconque des revendications 5 à 6, caractérisé en ce que la protéine est choisie parmi la caséine, l'élastine, le collagène, la kératine, la soie et les protéines de blé ou de soja.

8. Gel selon la revendication 5, caractérisé en ce qu'il comprend, en poids, 0,5-20 % de diméthicone copolyol ou de dérivés.

9. Gel selon l'une quelconque des revendications 5 ou 8, caractérisé en ce que les dérivés de diméthicone copolyol sont choisis parmi les diméthicones copolyols comportant un groupement phosphate, sulfate, chlorure de myristamide propyldiméthyl-ammonium, stéarate, amine ou glycomodifié.

10. Gel selon la revendication 5, caractérisé en ce qu'il comprend, en poids, 0,02-20 % d'un organopolysiloxane solubilisé par un tensioactif.

11. Gel selon l'une quelconque des revendications 5 ou 10, caractérisé en ce que l'organopolysiloxane solubilisé par un tensioactif est choisi parmi les polydiméthylsiloxanes et les mélanges de polydiméthylsiloxanes et de cyclométhicones.

12. Gel selon l'une quelconque des revendications 5, 10 ou 11, caractérisé en ce que l'organopolysiloxane solubilisé par un tensioactif se présente sous forme de microémulsion, la taille moyenne des particules d'organopolysiloxane étant de l'ordre de 1 µm.

13. Gel selon l'une quelconque des revendications précédentes, caractérisé en ce qu'il comprend, en poids, 0,01-30 % de polymère hydrophile.

14. Gel selon l'une quelconque des revendications précédentes, caractérisé en ce que le polymère hydrophile est choisi parmi les polymères ou copolymères acryliques, polyméthacryliques et/ou carboxyvinyliques; les dérivés de polysaccharides; les polymères de xanthane, de gellane, de rhamsan, les alginates, la maltodextrine, l'amidon et ses dérivés, l'acide hyaluronique et ses sels, la gomme de karoya, la farine de caroube, les dérivés de guar; les polyéthylèneglycols et leurs dérivés; les polyvinylpyrrolidones et leurs dérivés.

15. Gel selon l'une quelconque des revendications précédentes, caractérisé en ce qu'il comprend, en poids, 0,001-10 % de matière colorante.

16. Gel selon l'une quelconque des revendications précédentes, caractérisé en ce que la matière colorante est choisie parmi les colorants organiques et/ou les pigments, hydrophiles ou rendus hydrophiles.

17. Gel selon l'une quelconque des revendications précédentes, caractérisé en ce qu'il comprend, en poids, 0-30 % d'additif soluble ou dispersible dans l'eau.

18. Gel selon la revendication 17, caractérisé en ce que l'additif est choisi parmi les agents gélifiants autres qu'un polymère hydrophile, les actifs hydrosolubles, les agents neutralisants, les conservateurs hydrosolubles, les parfums, les charges et leurs mélanges.

19. Fond de teint consistant en un gel selon l'une quelconque des revendications précédentes.

20. Fard à joues ou à paupières consistant en un gel selon l'une quelconque des revendications 1 à 19.

21. Procédé pour maquiller le visage humain, caractérisé en ce qu'il consiste à appliquer sur le visage un gel conforme à l'une quelconque des revendications précédentes.

## Claims

1. Make-up gel comprising an agueous phase, a hydrophilic polymer as gelling agent and a colouring material which is soluble or dispersible in the agueous phase, characterized in that it comprises, in addition, at least one organopolysiloxane solubilized in the aqueous phase.

2. Gel according to Claim 1, characterized in that it comprises, by weight, 0.02-50 % by weight of organopolysiloxane solubilized in the agueous phase.

3. Gel according to either of the preceding claims, characterized in that it has an absolute viscosity with a value of 0.1-10 Pa.s (1-100 poises).

4. Gel according to any one of the preceding claims, characterized in that it has an absolute viscosity of 0.5-3 Pa.s (5-30 poises).

5. Gel according to any one of the preceding claims, characterized in that the organopolysiloxane solubilized in the agueous phase is chosen from the copolymers of siloxane and hydrolysed or unhydrolysed protein; dimethicone copolyols and their derivatives; and organopolysiloxanes solubilized using a surfactant of HLB (hydrophilic-lipophilic balance) value above 8.

6. Gel according to Claim 5, characterized in that it comprises, by weight, 1-50 % of a copolymer of siloxane and protein.

7. Gel according to either of Claims 5 and 6, characterized in that the protein is chosen from casein, elastin, collagen, keratin, silk and wheat or soya proteins.

8. Gel according to Claim 5, characterized in that it comprises, by weight, 0.5-20 % of dimethicone copolyol or derivatives.

9. Gel according to either of Claims 5 and 8, characterized in that the dimethicone copolyol derivatives are chosen from dimethicone copolyols containing a phosphate, sulphate, myristamidopropyldimethylammonium chloride, stearate, amine or glyco-modified group.

10. Gel according to Claim 5, characterized in that it comprises, by weight, 0.02-20 % of an organopolysiloxane solubilized with a surfactant.

11. Gel according to either of Claims 5 and 10, characterized in that the organopolysiloxane solubilized with a surfactant is chosen from polydimethylsiloxanes and mixtures of polydimethylsiloxanes and cyclomethicones.

12. Gel according to any one of Claims 5, 10 and 11, characterized in that the organopolysiloxane solubilized with a surfactant takes the form of a microemulsion, the average size of the particles of organopolysiloxane being of the order of 1 µm.

13. Gel according to any one of the preceding claims, characterized in that it comprises, by weight, 0.01-30 % of hydrophilic polymer.

14. Gel according to any one of the preceding claims, characterized in that the hydrophilic polymer is chosen from acrylic, polymethacrylic and/or carboxyvinyl polymers or copolymers; polysaccharide derivatives, xanthan, gellan and rhamsan polymers, alginates, maltodextrin, starch and its derivatives, hyaluronic acid and its salts, gum karaya, carob flour and guar derivatives; polyethylene glycols and their derivatives; and polyvinylpyrrolidones and their derivatives.

15. Gel according to any one of the preceding claims, characterized in that it comprises, by weight, 0.001-10 % of colouring material.

16. Gel according to any one of the preceding claims, characterized in that the colouring material is chosen from organic colorants and/or from pigments, which are hydrophilic or rendered hydrophilic.

17. Gel according to any one of the preceding claims, characterized in that it comprises, by weight, 0-30 % of a water-soluble or -dispersible additive.

18. Gel according to Claim 17, characterized in that the additive is chosen from gelling agents other than a hydrophilic polymer, water-soluble active agents, neutralizing agents, water-soluble preservatives, perfumes, fillers and mixtures thereof.

19. Make-up foundation, consisting of a gel according to any one of the preceding claims.

20. Blusher or eyeshadow, consisting of a gel according to any one of Claims 1 to 18.

21. Process for making up the human face, characterized in that it consists in applying to the face a gel according to any one of the preceding claims.

## Patentansprüche

1. Gel für Schminkzwecke, das eine wässerige Phase, ein hydrophiles Polymer als Gelbildner sowie ein in der wässerigen Phase lösliches oder dispergierbares Farbmittel enthält,
**dadurch gekennzeichnet, daß**
es ferner mindestens ein in der wässerigen Phase gelostes Polyorganosiloxan enthält.

2. Gel nach Anspruch 1, dadurch gekennzeichnet, daß es 0,02 bis 50 Gew.-% in der wässerigen Phase gelöstes Polyorganosiloxan enthält.

3. Gel nach einem der vorhergehenden Ansprüche, dadurch gekennzeichnet, daß es eine absolute Viskosität von 0,1 bis 10 Pa·s (1 bis 100 P) aufweist.

4. Gel nach einem der vorhergehenden Ansprüche, dadurch gekennzeichnet, daß es eine absolute Viskosität von 0,5 bis 3 Pa·s (5 bis 30 P) aufweist.

5. Gel nach einem der vorhergehenden Ansprüche, dadurch gekennzeichnet, daß das in der wässerigen Phase gelöste Polyorganosiloxan unter Copolymeren von Siloxanen mit hydrolysierten oder nichthydrolysierten Proteinen, Dimeticon-Copolyolen und ihren Derivaten und mit einem grenzflächenaktiven Mittel mit einem HLB-(hydrophilic lipophilic balance)-Wert von mehr als 8 solubilisierten Polyorganosiloxanen ausgewählt ist.

6. Gel nach Anspruch 5, dadurch gekennzeichnet, daß es 1 bis 50 Gew.-% eines Siloxan-Protein-Copolymers enthält.

7. Gel nach Anspruch 5 oder 6, dadurch gekennzeichnet, daß das Protein unter Casein, Elastin, Collagen, Keratin, Seide sowie Getreide- und Sojaproteinen ausgewählt ist.

8. Gel nach Anspruch 5, dadurch gekennzeichnet, daß es 0,5 bis 20 Gew.-% Dimeticon-Copolyol oder Derivate davon enthält.

9. Gel nach Anspruch 5 oder 8, dadurch gekennzeichnet, daß die Dimeticon-Copolyol-Derivate unter Dimeticon-Copolyolen ausgewählt sind, die eine Phosphatgruppe, eine Sulfatgruppe, eine N,N-Dimethyl-N-myristoylaminoPropylammoniumchlorid-Gruppe, eine Stearatgruppe, eine Aminogruppe oder eine glykomodifizierte Gruppe enthalten.

10. Gel nach Anspruch 5, dadurch gekennzeichnet, daß es 0,02 bis 20 Gew.-% eines mit einem grenzflächenaktiven Mittel solubilisierten Polyorganosiloxans enthält.

11. Gel nach Anspruch 5 oder 10, dadurch gekennzeichnet, daß das mit einem grenzflächenaktiven Mittel solubilisierte Polyorganosiloxan unter Polydimethylsiloxanen und Gemischen von Polydimethylsiloxanen und Cyclometiconen ausgewählt ist.

12. Gel nach einem der Ansprüche 5, 10 und 11, dadurch gekennzeichnet, daß das mit einem grenzflächenaktiven Mittel solubilisierte Polyorganosiloxan in Form einer Mikroemulsion vorliegt, in der die Polyorganosiloxanteilchen eine mittlere Teilchengröße von größenordnungsmäßig 1 µm aufweisen.

13. Gel nach einem der vorhergehenden Ansprüche, dadurch gekennzeichnet, daß es 0,01 bis 30 Gew.-% hydrophiles Polymer enthält.

14. Gel nach einem der vorhergehenden Ansprüche, dadurch gekennzeichnet, daß das hydrophile Polymer ausgewählt ist unter Acryl-, Polymethacryl- und/oder Carboxyvinyl-Polymeren oder -Copolymeren; Polysaccharidderivaten; Polymeren von Xanthan, Gellan, Ramsan, Alginaten, Maltodextrin, Stärke und ihren Derivaten, Hyaluronsäure und ihren Salzen, Karoyagummi, Johannisbrot-Kernmehl und Guarderivaten; Polyethylenglykolen und ihren Derivaten sowie Polvvinylpyrrolidonen und ihren Derivaten.

15. Gel nach einem der vorhergehenden Ansprüche, dadurch gekennzeichnet, daß es 0,001 bis 10 Gew.-% Farbmittel enthält.

16. Gel nach einem der vorhergehenden Ansprüche, dadurch gekennzeichnet, daß das Farbmittel unter hydrophilen oder hydrophil gemachten organischen Farbstoffen und/oder Pigmenten ausgewählt ist.

17. Gel nach einem der vorhergehenden Ansprüche, dadurch gekennzeichnet, daß es 0 bis 30 Gew.-% eines in Wasser löslichen oder dispergierbaren Additivs enthält.

18. Gel nach Anspruch 17, dadurch gekennzeichnet, daß das Additiv unter von hydrophilen Polymeren verschiedenen Gelbildungsmitteln, wasserlöslichen Wirkstoffen, Neutralisationsmitteln, wasserlöslichen Konservierungsmitteln, Parfums und Füllstoffen sowie Gemischen dieser Stoffe ausgewählt ist.

19. Make-up, das aus einem Gel nach einem der vorhergehenden Ansprüche besteht.

20. Wangenrouge oder Lidschatten, die aus einem Gel nach einem der Ansprüche 1 bis 19 bestehen.

21. Verfahren zum Schminken des menschlichen Gesichts, dadurch gekennzeichnet, daß es im Auftragen eines Gels nach einem der vorhergehenden Ansprüche auf das Gesicht besteht.
